# EUROPÄISCHE PATENTSCHRIFT

(11) **EP 0 307 654 B1**
(45) Veröffentlichungstag und Bekanntmachung des Hinweises auf die Patenterteilung: **12.01.1994**
(21) Anmeldenummer: 88113553.7
(22) Anmeldetag: 20.08.1988
(51) Int. Cl.: A61F 2/38

(54) **Implantat als Ersatz für einen resezierten Patellateil**
Implant as a substitute for a surgically removed patella part
Implant de substitution pour une partie de patella réséquée

(30) Priorität: 15.09.1987 DE 8712469 U
(43) Veröffentlichungstag der Anmeldung: 22.03.1989
(73) Patentinhaber: S + G IMPLANTS GMBH, D-23556 Lübeck (DE)
(72) Erfinder: Grundei, Hans, D-2400 Lübeck (DE); Thomas, Wolfram, Prof., D-2000 Hamburg 65 (DE); Scholz, Jörg, Dr., D-1000 Berlin 33 (DE)
(74) Vertreter: Eisenführ, Speiser & Partner

(56) Entgegenhaltungen:
- DE-A- 2 545 793
- DE-A- 2 608 628
- US-A- 4 158 894
- US-A- 4 344 192
- US-A- 4 353 135

## Beschreibung

Die Erfindung geht aus von einem bekannten Implantat als Ersatz für den rückwärtigen, durch einen ebenen Schnitt resezierten Patellateil nach den Merkmalen des Oberbegriffes des Anspruches 1. Wenn Kniegelenk-Endoprothesen implantiert werden, muß die Patella rückseitig reseziert und durch ein Implantat aus einem geeigneten Kunststoff ersetzt werden, denn die Rückseite der natürlichen Patella würde zu einem sehr schnellen Abrieb an den Flächen der Kniegelenk-Endoprothese führen. Es muß daher der rückwärtige resezierte Teil der Patella durch ein entsprechendes Implantat der eingangs erwähnten Art ersetzt werden.

Die Aufgabe der Erfindung besteht darin, das Implantat fester und sicherer als bisher auf der Rückseite der resezierten Patella zu verankern.

Diese Aufgabe wird bei einem Implantat gemäß dem Oberbegriff des Hauptanspruches erfindungsgemäß dadurch gelöst, daß der Zapfen bei Anlage der Zentrierscheibe gegen die Implantatfläche mit dem konischen Ende, welches mit dem Implantat verbindbar ist, neben dem Kraftschluß durch Formschluß in der Einbohrung des Implantats sitzt, und daß die metallische, auf dem Zapfen befestigte Zentrierscheibe ausschließlich in ihrem radial äußeren Umfangsbereich offenzellig ausgebildet ist.

Durch den zusätzlichen Formschluß zwischen dem Zapfen und dem Implantat und durch die offenzellige Ausbildung der Umfangsfläche der Zentrierscheibe wird eine verbesserte Verankerung des Implantats an der restlichen Patella verwirklicht, weil nämlich Knochengewebe in die Zentrierscheibe einwachsen, und das Implantat über den Zapfen kraft- und formschlüssig an der Patella verankert werden kann.

Vorteilhaft geht man dabei so vor, daß das in die Einbohrung des Implantats eingreifende konische Ende des Zapfens mit mindestens einer Umfangsnut in Winkelform versehen ist, deren nach rückwärts gerichtete Nutwandung senkrecht zur Zapfenachse liegt und daß das konische Zapfenende einen größeren Durchmesser als die Einbohrung besitzt. Dadurch wird neben dem bekannten konischen Kraftschluß zwischen den Zapfen und dem Implantat zusätzlich ein Formschluß erreicht, da ein Teil des Implantatmaterials in die Umfangsnut oder in die Umfangsnuten eindringt.

Die Neuerung wird nachstehend anhand der Zeichnung beschrieben. Es zeigt:
- Figur 1: einen Schnitt durch das Implantat nach der Neuerung mit angedeutetem, resezierten Patellateil,
- Figur 2: eine Abänderung des Befestigungszapfens für das Implantat, und
- Figur 3: einen Schnitt durch den mit X angedeuteten Teil des Befestigungszapfens.

Beim Implantieren einer Kniegelenk-Endoprothese muß der rückwärtige Teil der Patella 1 durch einen ebenen, gegebenenfalls auch abgestuften Schnitt reseziert und durch ein den resezierten Teil ersetzendes Implantat aus einem geeigneten Kunststoff ersetzt werden. Zu diesem Zweck wird das Implantat 3 mit einer mittigen Einbohrung 4 auf der ebenen abgestuften Fläche 3a versehen. In die Abstufung legt sich eine metallische Zentrierscheibe 5, die auf dem Zapfen 6 befestigt ist. Die Zentrierscheibe 5 ist auf dem Umfang oder insgesamt aus einem offenzelligen Metall hergestellt, um dadurch eine verbesserte Verbindung mit der Patella 1 zu erreichen.

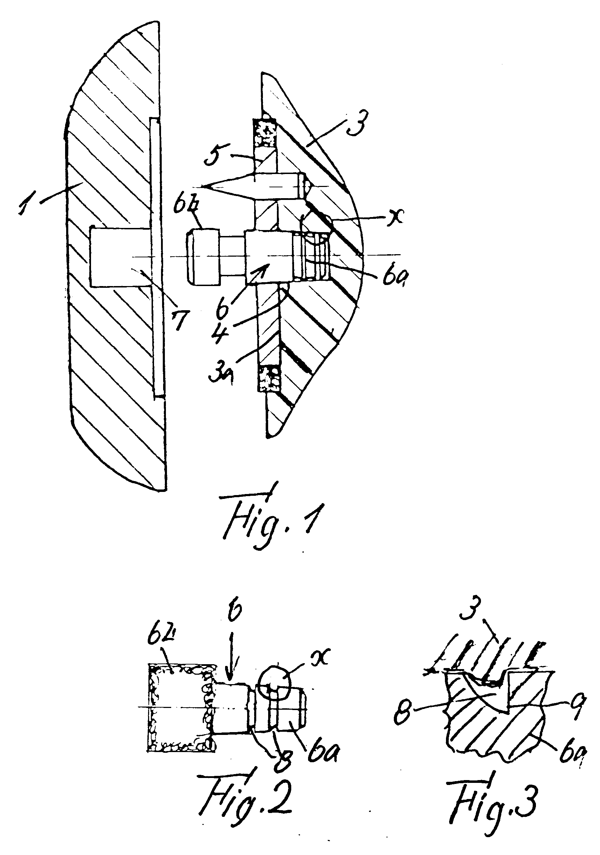

Der Zapfen 6 ist für den Eingriff in die Bohrung 4 des Implantats 3 mit einem konischen, selbsthemmenden Ende versehen und greift beim Verbinden mit der Patella mit dem anderen Ende 6b in eine Ausnehmung 7 der Patella und wird hier einzementiert. Das Ende 6b kann nach Figur 2 ebenfalls offenzellig ausgebildet werden, wobei dann ein Einzementieren in der Ausnehmung 7 nicht mehr erforderlich ist.

Gemäß der Erfindung wird neben dem Kraftschluß durch den Eingriff des konischen Zapfenendes 6a in die Bohrung 4 zusätzlich ein Formschluß erreicht, durch den die Verbindung des Zapfens 6 mit dem Implantat 3 wesentlich verbessert wird. Zu diesem Zweck ist das Zapfenende 6a mit mindestens einer Umfangsnut 8, z.B zwei Nuten 8 mit einem Querschnitt in Winkelform versehen, wie Figur 3 zeigt. Dabei liegt die nach rückwärts gerichtete Nutwandung 9 senkrecht zur Zapfenachse und der Durchmesser des Zapfenendes 6a ist größer gewählt als die Einbohrung 4, so daß beim Eindrücken des Zapfenendes in die Einbohrung 4 Material des Implantats 3 in die Nut 8 eingedrückt wird, wie die Figur 3 zeigt. Damit ist zusätzlich ein Formschluß zwischen dem Zapfen 6 und dem Implantat erreicht.

## Patentansprüche

1. Implantat als Ersatz für den rückwärtigen, durch einen ebenen oder abgestuften Schnitt resezierten, einer Kniegelenk-Endoprothese zugekehrten Patellateil, bestehend aus einem dem resezierten Patellateil nachgebildeten Kunststoffteil (3), in dessen mittiger Einbohrung (4) auf der ebenen, abgestuften Implantatfläche (3a) das eine Ende eines eine zentrierende, sich an die ebene Fläche anlegende Metallscheibe (5) tragenden metallischen, konischen Zapfens (6) selbsthemmend befestigt ist, der auf der anderen Scheibenseite ein in einer Bohrung (7) der Patella (1) zu zementierendes Ende besitzt, dadurch gekennzeichnet, daß der Zapfen (6) bei Anlage der Zentrierscheibe (5) gegen die Implantatfläche (3a) mit dem konischen Ende (6a), welches mit dem Implantat verbindbar ist, neben dem Kraftschluß durch Formschluß in der Einbohrung (4) des Implantats (3) sitzt, und daß die metallische, auf dem Zapfen (6) befestigte Zentrierscheibe (5) ausschließlich in ihrem radial äußeren Umfangsbereich offenzellig ausgebildet ist.

2. Implantat nach Anspruch 1,
dadurch gekennzeichnet, daß das in die Einbohrung (4) des Implantats (3) eingreifende, konische Ende (6a) des Zapfens (6) mit mindestens einer Umfangsnut (8) in Winkelform versehen ist, deren rückwärts gerichtete Nutwandung (9) senkrecht zur Zapfenachse liegt und daß das konische Zapfenende (6a) einen größeren Durchmesser als die Einbohrung (4) besitzt.

3. Implantat nach einem der Ansprüche 1 oder 2,
dadurch gekennzeichnet, daß das Zapfenende (6b) für den Eingriff in die Patella (1) offenzellig ausgebildet ist.

## Claims

1. An implant as a substitute for the rear patella part, which is surgically removed by a plane or stepped cut and is turned towards a knee joint endoprosthesis, consisting of a plastic part (3) imitating the surgically removed patella part, in the central bore (4) of which on the plane, stepped implant surface (3a) is attached in a self-locking manner one end of a metal, conical peg (6) bearing a centring metal disk (5), lying against the plane surface, which peg on the other side of the disk possesses an end to be cemented into a bore (7) in the patella (1),
**characterised in that,** when the centring disk (5) abuts against the implant face (3a), with the conical end (6a), which can be connected to the implant, the peg (6) is seated in the bore (4) of the implant (3) with force locking as well as with form fit,
**and in that** the metal centring disk (5) attached to the peg (6) is designed with open cells exclusively in the radially outer peripheral region.

2. An implant according to Claim 1,
**characterised in that** the conical end (6a) of the peg (6) engaging into the bore (4) of the implant (3) is provided with at least one peripheral groove (8) of angular shape, the rearwardly directed groove wall (9) of which lies perpendicular to the peg axis, **and in that** the conical peg end (6a) possesses a larger diameter than the bore (4).

3. An implant according to one of Claims 1 or 2,
**characterised in that** the peg end (6b) for engagement in the patella (1) is constructed with open cells.

## Revendications

1. Implant de substitution pour la partie arrière d'une rotule réséquée par une couple plane ou à gradin, cette partie étant dirigée vers une endoprothèse d'une articulation de genou, constitué d'un élément en matière plastique (3) reproduisant la partie de rotule réséquée, cet élément présentant, sur la face d'implant plane, à gradin (3a), un alésage de montage central (4) dans lequel est fixé par autoblocage l'une des extrémités d'une cheville conique (6) portant un disque métallique de centrage (5) qui repose sur la face plane, cette cheville comportant, de l'autre côté du disque, une extrémité devant être scellée dans un alésage (7) de la rotule (1), caractérisé en ce que la cheville (6) est, lorsque le disque de centrage (5) vient en contact avec la face d'implant (3a), montée dans l'alésage de montage (4) de l'implant (3) par l'extrémité conique (6a) de liaison avec l'implant, non seulement par liaison par force, mais aussi par liaison géométrique, et en ce que le disque métallique de centrage (5) fixé sur la cheville (6) est constitué à alvéoles ouverts exclusivement dans sa zone périphérique extérieure en direction radiale.

2. Implant selon la revendication 1, caractérisé en ce que l'extrémité conique (6a) de la cheville (6) s'engageant dans l'alésage de montage (4) de l'implant (3) est munie d'au moins une rainure périphérique (8) de forme angulaire, la paroi (9) de cette rainure située en arrière étant perpendiculaire à l'axe de la cheville, et en ce que l'extrémité conique (6a) de la cheville présente un diamètre plus grand que celui de l'alésage de montage (4).

3. Implant selon l'une des revendications 1 et 2, caractérisé en ce que l'extrémité (6b) de la cheville est constituée à alvéoles ouverts pour venir en prise dans la rotule (1).
